Europäisches Patentamt

European Patent Office

Office européen des brevets

(19)

(11) Veröffentlichungsnummer: **0 213 469**
**A2**

(12) **EUROPÄISCHE PATENTANMELDUNG**

(21) Anmeldenummer: 86111079.9

(51) Int. Cl.⁴: **A61K 35/14**

(22) Anmeldetag: 11.08.86

(30) Priorität: 03.09.85 DE 3531430

(43) Veröffentlichungstag der Anmeldung:
**11.03.87 Patentblatt 87/11**

(84) Benannte Vertragsstaaten:
**BE DE FR GB IT LU NL SE**

(71) Anmelder: **Biotest Pharma GmbH**
**Flughafenstrasse 4**
**D-6000 Frankfurt 71(DE)**

(72) Erfinder: **Gänshirt, Karl-Heinz, Dr. Dipl.-Chem.**
**August-Bebel-Strasse 34**
**D-6072 Dreieich(DE)**
Erfinder: **Handel, Klaus-Dieter, Dr. Dipl.-Chem.**
**Palleswiesenstrasse 14**
**D-6100 Darmstadt(DE)**
Erfinder: **Walker, Wolfram H., Dr. Dipl.Chem.**
**Thomastrasse 8**
**D-6074 Rödermark(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**D-6230 Frankfurt am Main 80(DE)**

(54) Verfahren zur Gewinnung von Komponenten aus einer Flüssigkeit über räumlich getrennte Bereiche im Blutbeutelsystem, sowie Vorrichtung zur Durchführung des Verfahrens.

(57) Ein Verfahren zur Gewinnung von Komponenten aus einer Flüssigkeit über räumlich getrennte Bereiche im Beutelsystem bei dem man einen mit dieser Mehrkomponenten-Flüssigkeit gefüllten Beutel zwischen mindestens ein Trennelement hängt und das Trennelement in eine Höhe mit der Grenzschicht bringt und anschließend in Abquetschposition bringt und die Beutelfüllung in getrennte flüssigkeitsdichte Volumina teilt, läßt sich dahingehend verbessern, daß es einen dauernden Einsatz ermöglicht bei sicherer und sehr genauer Trennung der Komponenten, indem man eine Maßzahl, die die Höhe der Grenzschicht angibt, ermittelt, die Höhe des Abquetschens mit Hilfe des Trennelementes anhand der ermittelten Maßzahl der Höhe der Grenzschicht im Beutel sowie der Beutelgröße und dessen Füllvolumen ermittelt, die Höheneinstellung des Trennelementes oder des Beutels mit Hilfe einer Energiequelle vornimmt und dann mit Hilfe einer Energiequelle das Trennelement in Abquet-schposition bringt.

Fig. 1

## Verfahren zur Gewinnung von Komponenten aus einer Flüssigkeit über räumlich getrennte Bereiche im Blutbeutelsystem, sowie Vorrichtung zur Durchführung des Verfahrens

Die Erfindung betrifft ein Verfahren zur Gewinnung von Komponenten aus einer Flüssigkeit in der die Komponenten in mindestens zwei räumlich getrennten Bereichen vorliegen nach dem Oberbegriff des Anspruchs 1 und Vorrichtung zur Durchführung des Verfahrens nach dem Oberbegriff des Anspruchs 13, sowie Hilfsmittel zur Durchführung des Verfahrens.

Zum Zwecke der Transfusion oder Transplantation ist es häufig erforderlich, die zu transfundierenden oder transplantierenden Mehrkomponenten-Flüssigkeiten in einzelne Komponenten aufzutrennen bzw. bestimmte Zellen in gereinigter und/oder konzentrierter Form zu erhalten.

So reagieren bei der Bluttransfusion polytransfundierte Patienten häufig mit der Bildung von Antikörpern, die eine Effizienz späterer Bluttransfusionen erheblich kompromittieren. Insbesondere störend wirken sich sogenannte HLA-Antikörper aus, die vorwiegend gegen Leukozyten bzw. Thrombozyten gerichtet sind. Bei erforderlichen Transfusionen von Erythrozytensedimenten muß daher getrachtet werden, durch präparative Maßnahmen diese möglichst vollständig von HLA-Antigen-Trägern zu befreien. Hierzu wurden bisher zahlreiche Techniken vorgeschlagen, die jedoch sehr zeit-und personalintensiv sind und zum Teil tiefgreifende Manipulationen des Blutes erfordern - (vgl. Med. Welt 30 (1979) Nr. 43, S. 1597-1598).

Aus der US-PS 3,064,647 bzw. 3,986,506 sind Blutbeutelsysteme bekannt, die aus mindestens 3 Einzelbeuteln bestehen, bei denen Blut in einen Beutel abgenommen wird und die Blutkomponenten sowohl am Beuteloberteil oder Kopfteil als auch am Beutelunterteil oder Bodenteil abgetrennt werden können.

In der DE-OS 30 12 227 wird ein Verfahren und Blutbeutelsystem zur Trennung von Blutkomponenten beschrieben, bei dem Blut im Blutbeutel gesammelt wird, der einen Auslaß am Kopf und Boden besitzt, der Blutbeutel zentrifugiert wird und das Plasma am Oberteil des Beutels, das Erythrozytenkonzentrat am Unterteil des Beutels und die Zwischenschicht (buffy coat) sich im Beutel befindet. Die Geschwindigkeit des Ablaufs des Plasmas und Erythrozytenkonzentrates wird so gesteuert, daß sich das buffy coat in einen bestimmten Bereich des ursprünglichen Beutels befindet.

In der DE-OS 30 12 228 wird eine Vorrichtung zur Bluttrennung durch Ausdrücken beschrieben unter Zuhilfenahme eines Blutzelldetektorsystems.

Bei den vorstehend genannten Systemen erfolgt die Trennung der Blutkomponenten durch Abdrücken oder Absaugen, wodurch Durchmischungen der einzelnen Komponenten an den Grenzschichten erfolgen. Die Qualität und die Quantität der so gewonnenen Blutkomponenten entspricht somit nicht den Anforderungen wie sie gemäß aaO Med. Welt dargelegt werden.

Ein für die Trennung von Erythrozytensedimenten von leukozyten-und thrombozytenreichen Schichten besonders geeignetes Verfahren und Gerät zu seiner Durchführung werden aaO Med. Welt S. 1958-1601 beschrieben.

Der Leitgedanke des dort beschriebenen Verfahrens besteht darin, daß die Trennung der nach kopfstehender Zentrifugation gewonnenen Erythrozytensedimente von den leukozyten-thrombozytenreichen Schichten (Buffy-coat) durch eine gezielte und absolut flüssigkeitsdichte Unterteilung des Blutes in zwei Komponenten erfolgt. Der Inhalt der unteren Kammer (leukozyten-thrombozytenarme Ery-Sedimente) kann ohne jede Verwirbelung und Vermischung, wie sie sonst beim Abpressen oder Absaugen des Buffy coats entsteht, in einem Satellitenbeutel zur Transfusion abgeleitet werden.

Zur Durchführung des Verfahrens wird dort ein Gerät beschrieben, das ein in der Höhe verstellbares Trenngestell mit horizontal zusammen-bzw. auseinanderfahrbaren Trennbacken und eine Aufhängung für den Beutel mit der in Komponenten zu trennenden Flüssigkeit enthält.

Sowohl die Höheneinstellung als auch das Zusammenfahren der beiden Trennbacken erfolgt dort manuell. Dies erfordert einigen Kraftaufwand, der bei dauernder Benutzung ermüdend ist,sowie erheblichen Zeitaufwand.Hinzu kommt, daß das Präparationsergebnis von der Arbeitsweise der durchführenden Person bestimmt wird. Die Ermittlung der Grenzschicht und vor allem das Ansteigen der Grenzschicht, das vom Gegendruck der Hand auf die obere Beutelhälfte abhängt, beim Zusammenfahren der Trennbacken sind individuell beeinflußt.

Der Erfindung liegt die Aufgabe zugrunde, das vorstehend beschriebene und gattungsgemäße Verfahren derart zu verbessern, daß es einen dauernden Einsatz ermöglicht bei sicherer und sehr genauer Trennung der Komponenten, sowie eine Vorrichtung und Hilfsmittel bereitzustellen, mit der dieses vorteilhafte Verfahren durchführbar ist.

Diese Aufgabe wird bei einem gattungsgemäßen Verfahren durch die kennzeichnenden Merkmale des Anspruchs 1 und bei einer gattungsgemäßen Vorrichtung durch die kennzeichnenden Merkmale des Anspruchs 9 gelöst.

Die Ermittlung der Maßzahl, die die Höhe der Grenzschicht angibt und die anhand dieser ermittelten Maßzahl, der Beutelgröße und dessen Füllvolumen ermittelte Höheneinstellung des Trennelementes bzw. des Beutels, die in der Regel eine andere ist als die Höhe der Grenzschicht, weil sich beim Zusammenquetschen des Beutels letztere verschiebt, ermöglicht optimale Abklemmung an der Phasengrenze und damit eine sehr exakte Trennung der Komponenten in den einzelnen Schichten. Es wird dabei sowohl vermieden, daß unerwünschte Komponenten der einen Schicht mit in die die zu gewinnende Schicht übergehen und somit das Therapieergebnis verschlechtern, als auch daß erwünschte Komponenten einer Schicht zurückbleiben, wodurch die Ausbeute dieser Komponenten verringert wird.

Durch die Vertikal-und/oder Horizontaleinstellung der Trennelemente oder Vertikaleinstellung des Beutels mit Hilfe von Energiequellen, vorzugsweise Motoren entfällt der Kraftaufwand einer manuellen Bedienung, und das Verfahrn kann kontinuierlich durchgeführt werden.

Bei einer Ausführungsform des erfindungsgemäßen Verfahrens zur Gewinnung einer Blutkomponente und unter Verwendung von einem in der Höhe verstellbaren Paar Trennbacken wird ein zentrifugierter Blutbeutel aus einem für Blutbeutel üblichen durchsichtigen Kunststoff vorsichtig im Gerät aufgehängt, damit die Grenzschicht zwischen dem Erythrozyten-Sediment und dem Plasma nicht aufgewirbelt wird.

Es wird zunächst eine Maßzahl für die Höhe der Grenzschicht ermittelt. Hierzu bringt man die Trennbacken auf die Höhe der Grenzschicht.

Durch Betätigen der Schalter im Steuergerät wird der Motor zur Einstellung der Höhe der Trennbacken gesteuert. Dieser Motor treibt eine vertikale Spindel an, die die Halterung für die Trennbacken anhebt oder absenkt. Der Motor kann seitlich oder unterhalb der Spindel angebracht sein.

Das Einstellen der genauen Höhe kann erleichtert werden, indem auf den Trennbacken eine Lichtquelle montiert ist, die einen Lichtpunkt auf die Beuteloberfläche projiziert. Die Trennbacken werden in der Höhe so lange verstellt, bis der Lichtpunkt mit der Grenzschicht übereinstimmt.

Eine besonders zweckmäßige Ausführungsform besteht in der Ausleuchtung des Plasmas mit einer Lichtquelle, die auf einer der Trennbacken befestigt ist. Ein optischer Detektor ist gleichzeitig an der Unterseite der Trennbacke angebracht. Fährt die Trennbacke mit der Lichtquelle und dem optischen Detektor an der Grenzschicht vorbei, so wird vom optischen Detektor eine Änderung der Helligkeit aufgrund der Grenzschicht festgestellt. Die Höhe der Helligkeitsänderung ist ein Maß für die Höhe der Grenzschicht.

Optische Detektoren durchstrahlen normalerweise ein System, d.h. zwischen der Lichtquelle und dem optischen Detektor ist das zu untersuchende Gut angebracht. Anhand der Lichtdurchlässigkeit wird zwischen verschiedenen Schichten unterschieden. Bei Blutbeuteln, die mit Etiketten versehen sind, wirken sich letztere störend auf das Transparenz-Kriterium aus. Zweckmäßigerweise sollte bei dieser Ausführungsform deshalb die Einstrahlung des Lichtes von der gleichen Seite erfolgen auf der der optische Detektor sich befindet. Außerdem sollte der Beutel so aufgehängt werde, daß seine nicht etikettierte Rückseite der Lichtquelle und dem Detektor zugewandt sind.

Man stellte außerdem fest, daß eine Fokussierung des Lichtstrahles auf die Beutelrückwand nicht zur Erkennung der Grenzschicht führt. Das Licht, das der optische Detektor registrierte, wurde zumeist durch die Oberfläche des Beutels reflektiert. Je nach dem Füllvolumen der Beutel wurde das Licht verschieden reflektiert, so daß eine Justierung der Empfindlichkeit nicht möglich war.

Das Einstrahlen von Licht, das nicht fokussiert ist, brachte überraschenderweise das gewünchte Ergebnis. Ein Großteil des eingestrahlten Lichtes wird an der gegenüberliegenden Etikettenrückseite sowie infolge der Trübung des Plasmas diffus zurückgestreut. Wenn der optische Detektor an der nicht etikettierten Beutelwand vertikal entlanggeführt wird, empfängt er plötzlich weniger Licht, sobald er sich etwas unterhalb der Grenzschicht befindet. Die Grenzschicht kann hiermit reproduzierbar erkannt werden.

Das Ablesen der Maßzahl für die Höhe erfolgt über einen Zeiger, der an den Trennbacken befestigt ist und an einer am Gerät fixierten Skala oder Nomogrann entlangfährt. Ebenso kann von einer Grundeinstellung aus die Höhe aufgrund der Umdrehungen des Elektromotors bzw. der Vertikalspindel ermittelt werden.

Aus der Maßzahl für die Höhe der Grenzschicht wird die Höhe der Trennbacken für das Abquetschen ermittelt. Diese Korrektur ist von der Beutelgestalt, Beutelgröße, Füllvolumen der Beutel und der Höhe der Grenzschicht im Beutel abhängig. Diese Korrekturen werden empirisch ermittelt.

Die so ermittelten Korrekturen können tabelliert werden. Vorzugsweise können die Tabellenwerte auf ein Nomogramm übertragen werden. Auf dem Nomogramm ist dann aus der Maßzahl für die Höhe direkt die Höhe der Trennbacken, bei der diese zusammenfahren müssen, abzulesen.

Dieses Nomogramm kann auch anstelle der Skala direkt am Gerät angebracht sein. Da in Abhängigkeit der Beutelgröße und dessen Füllvolumen verschiedene Nomogramme vorliegen, ist es zweckmäßig, wenn eine Halterung vorliegt, in der die Nomogramme leicht auswechselbar sind. Die Aufgabe des Nomogramms kann jedoch auch ein Rechner übernehmen.

Die Trennbacken werden auf diese ermittelte Höhe nachgefahren. Zur Erleichterung dieses Vorganges kann auch ein verschiebbarer Endschalter benutzt werden. Der Endschalter wird auf die ermittelte Trennhöhe eingestellt. Die Trennbacken läßt man dann in vertikaler Richtung auf diesen Schalter zufahren. Sobald der eingestellte Punkt erreicht ist, bleiben sie stehen und fahren zusammen.

Der Endschalter kann auf mechanische, optische oder induktive Weise arbeiten.

Das Zusammenfahren der Trennbacken erfolgt ebenfalls mit einem Elektromotor, der durch Betätigen des entsprechenden Schalters im Steuergerät ein-bzw. ausgeschaltet wird. Dieser Motor befindet sich auf der Halterung, die auch die Trennbacken aufnimmt, und bewegt sich bei der Höheneinstellung der Trennbacken mit. Er treibt eine Spindel an, die über zwei gegenläufige Steigungen die beiden Trennbacken zusammen-bzw. auseinanderzieht.

Die Schalter für die Elektromotoren befinden sich zweckmäßigerweise an der Front des Steuergerätes unterhalb der Vorrichtung. Es ist mindestens jeweils ein Schalter für die Auf-und Abwärtsbewegung sowie für das Zusammen-und Auseinanderfahren der Trennbacken vorgesehen. Aus Sicherheitsgründen sind noch Endschalter vorgesehen, die den oberen und unteren Anschlag sowie die geschlossene und maximal geöffnete Position der Trennbacken melden und die Motoren dann abschalten. Zweckmäßigerweise wird noch ein Stop-Schalter angebracht, um jederzeit die Motoren unterbrechen zu können.

Nach dem Abquetschen des Beutels kann die gewünschte Komponente dem Beutel entnommen werden, ohne daß eine Vermischung der beiden zu trennenden Volumina erfolgt. Nach der Abtrennung werden die Trennbacken wieder in maximal geöffnete Positionen gefahren und der Beutel aus dem Gerät herausgenommen.

Der gesamte Ablauf des Verfahrens kann auch automatisch gestaltet werden. Hierzu ist es notwendig, daß die Höhe der Trennbacken in ein elektrisches Signal verwandelt wird. Zu diesem Zweck kann, wie schon beschrieben, die Höhe der Trennbacken aus der Umdrehungszahl des Motors bzw. der Spindel laufend registriert werden. Dieses Signal dient dann als Eingangssignal für ein automatisches Steuergerät. Nach Drücken eines Start-Schalters ermittelt es zunächst mit Hilfe des optischen Detektors die Höhe der Grenzschicht. Hieraus berechnet es die Korrektur und fährt die Trennbacken nach. Anschließend läßt es die Trennbacken zusammenfahren. Nach der Präparation des Blutbeutels läßt es die Trennbacken nach Drücken eines Schalters wieder auseinanderfahren.

Ein leukozyten-thrombozytenarmes Erythrozytensediment läßt sich nach dem erfindungsgemäßen Verfahren z.B. dadurch gewinnen, daß man eine kopfstehend stark zentrifugierte Blutkonserve kopfstehend in die erfindungsgemäße Vorrichtung hängt.

Durch Ansetzen der Trennbacken unterhalb des Buffy coats kann ein leukocyten-und thrombozytenarmes Erythrozytenkonzentrat vom Plasma und Buffy coat, das die Leukozyten und Thrombozyten enthält, abgetrennt werden.

Durch schwaches Zentrifugieren kann man ein leukozytenarmes thrombozytenreiches Plasma gewinnen, während die Leukozyten sich im Buffy coat befinden. Durch Ansetzen der Trennbacken oberhalb des Buffy coats läßt sich mit Hilfe des erfindungsgemäßen Verfahrens leukozytenarmes thrombozytenreiches Plasma abtrennen, das zu Thrombozytenkonzentrat aufgearbeitet werden kann.

Das erfindungsgemäße Verfahren läßt sich jedoch auch mit zwei oder mehreren Trennelementen durchführen, je nachdem wieviel unterschiedliche Schichten in einem Beutel voneinander getrennt werden sollen.

So läßt sich z.B. Blut in die drei Blutkomponenten Plasma, buffy coat bzw. Thrombozytenkonzentrat und Erythrozytenkonzentrat durch Verwendung von zwei untereinanderangeordneten Trennelementen trennen.

Zur Trennung in drei Komponenten verwendet man vorzugsweise ein Blutbeutelsystem mit Oben-und Untenabgang, das aus einem Blutentnahmebeutel mit Blutstabilisatorlösung mit integriertem Blutentnahmesystem besteht, dessen Längsseite zur Schmalseite im Verhältnis ca. 2 : 1 ist und der auf der einen Schmalseite einen Abgang zu einem Leerbeutel bzw. zu einem Beutel mit Thrombozytenkonservierungslösung und auf der gegenüberliegenden Schmalseite einen Abgang zu einem Beutel besitzt, der eine Erythrozytenkonservierungslösung enthält. Der Abgang zu den Beuteln ist mit einem Abbrechteil verschlossen. Der Beutel enthält außerdem einen Stutzen zum Einstechen eines Einstichdorns.

Mit diesem Beutelsystem lassen sich die drei Blutkomponenten derart trennen, daß nach der Zentrifugation des mit Blut gefüllten Entnahmebeutels der Beutel entlang der Schmalseite im Bereich der Grenzschicht zwischen dem unteren Erythrozytenkonzentrat und dem mit buffy coat durch mindestens ein Trennelement abgeklemmt wird, anschließend das Erythrozytenkonzentrat in den Beutel mit der Erythrozytenkonservierungslösung eingeleitet wird, das obere Plasma in den leeren Beutel abgepreßt wird und das buffy coat im ursprünglichen Blutentnahmebeutel verbleibt. Das Thrombozytenkonzentrat wird anschließend aus dem buffy coat gewonnen und mit einer Thrombozytenkonservierungslösung versetzt. Die Menge und das Volumen des im Blutentnahmebeutel verbleibenden buffy coats wird zum einen durch die Position des Trennelementes zwischen buffy coat und Erythorzytenkonzentrat und zum anderen durch die Menge bzw. das Volumen des abgetrennten Plasmas festgelegt und gesteuert.

Vorzugsweise kann die Steuerung der Menge des nach oben abgepreßten Plasmas durch eine Photozelle erfolgen. Die Photozelle kann so ausgelegt werden, daß z.B. bei Erscheinen bestimmter Mengen des buffy coats, was sich in einer Verminderung der Transparenz des Plasmas zeigt, der Abfluß des Plasmas unterbrochen wird. Ein für diesen Zweck besonders geeignetes Photozellensystem wird in der Firmenzeitschrift Melco Engineering Corporation, "Cell Sensor", Januar 1983, Form # 4 beschrieben.

Die Entnahmegeschwindigkeit der durch Abquetschen getrennten Komponenten aus dem Beutel kann durch Abpressen beschleunigt werden.

Das erfindungsgemäße Verfahren läßt sich auch bei anderen zellenhaltigen Flüssigkeiten außer Blut anwenden. Beispielsweise eignet es sich zur Zellreinigung bei Knochentransplantationen.

Darüberhinaus lassen sich auch andere Flüssigkeiten mit in räumlich getrennten Bereichen vorliegenden Komponenten auftrennen.

Nachstehende Zeichnungen dienen der weiteren Erläuterung der erfindungsgemäßen Vorrichtung.

Es zeigen

Fig. 1 eine perspektivische Ansicht einer Ausführungsform der erfindungsgemäßen Vorrichtung ohne Träger für eine Skala oder ein Nomogramm.

Fig. 2 eine perspektivische Ansicht einer Ausführungsform der erfindungsgemäßen Vorrichtung mit Träger für eine Skala oder ein Nomogramm;

Fig. 3 eine Darstellung zur Erläuterung der Funktion des Nomogramms;

Fig. 4 A bis D die Seitenansicht verschiedener Versionen, eines Abquetschsystems in Kombination mit Abpreßvorrichtungen für den gefüllten Beutel, wobei in den Fig. 4 B bis D der Übersichtlichkeit halber der Beutel weggelassen wurde.

Fig. 4 E einen Aufriß eines Abquetschsystems mit einer besonders ausgebildeten Beutelaufnahmefläche.

Fig. 5 A und B für das erfindungsgemäße Verfahren geeignete Beutelsysteme;

Fig. 6 A und B für das erfindungsgemäße Verfahren besonders geeignete Blutbeutel.

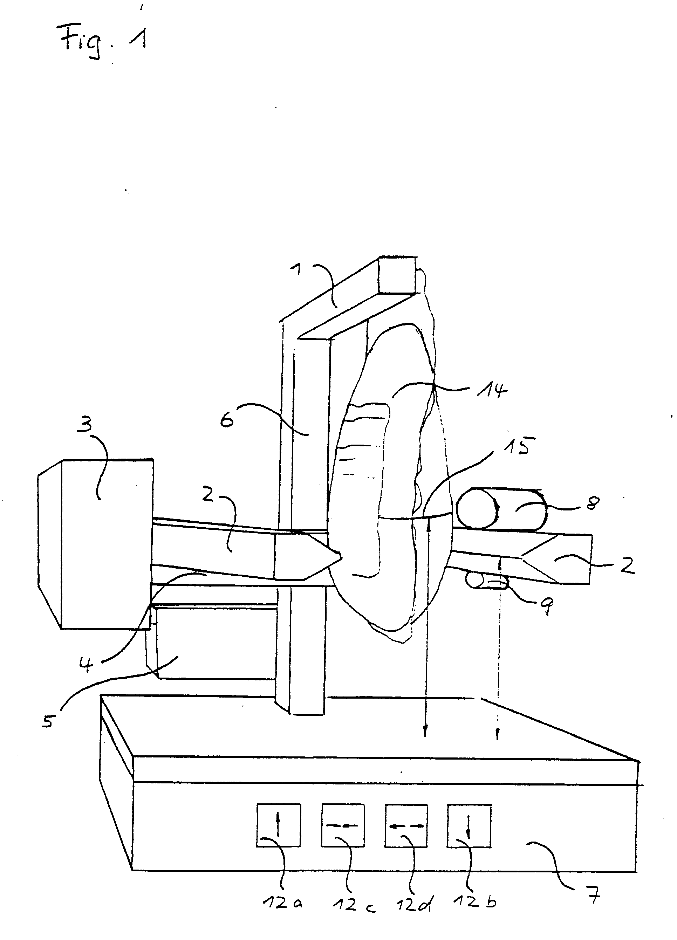

In Fig. 1 bedeutet (1) eine Aufhängung für den Beutel (14). Trennbacken (2) und Motor (3) zum horizontalen Zusammen-bzw. Auseinanderfahren der Trennbacken befinden sich an einer Halterung - (4). Motor (5) zur Höheneinstellung der Trennbacken befindet sich an einer Halterung (6). In dieser Ausführungsform befindet sich eine Lichtquelle (8) auf der der Beutelrückseite zugekehrten Trennbacke und ein optischer Detektor (9) an der gleichen Trennbacke unterhalb der Lichtquelle bzw. unterhalb des Abquetschteils der Trennbacke. Es ist jedoch auch eine Ausführungsform möglich, die einen fokussierten Lichtzeiger als Lichtquelle (8) enthält, wobei der Detektor entfällt. Die ganze Anlage ist durch Halterung (6) mit einem Steuergerät (7) verbunden. An der Frontseite des Steuergerätes (7) befinden sich die Schalter (12a, 12b) für Motor - (5) zur Höheneinstellung der Trennbacken und Schalter (12c, 12d) für Motor (3) zum Zusammen- und Auseinanderfahren der Trennbacken. Die beiden Pfeile rechts oberhalb des Steuergeräts zeigen die unterschiedliche Höheneinstellung der Grenzschicht (15) und der Trennbacken (2) an.

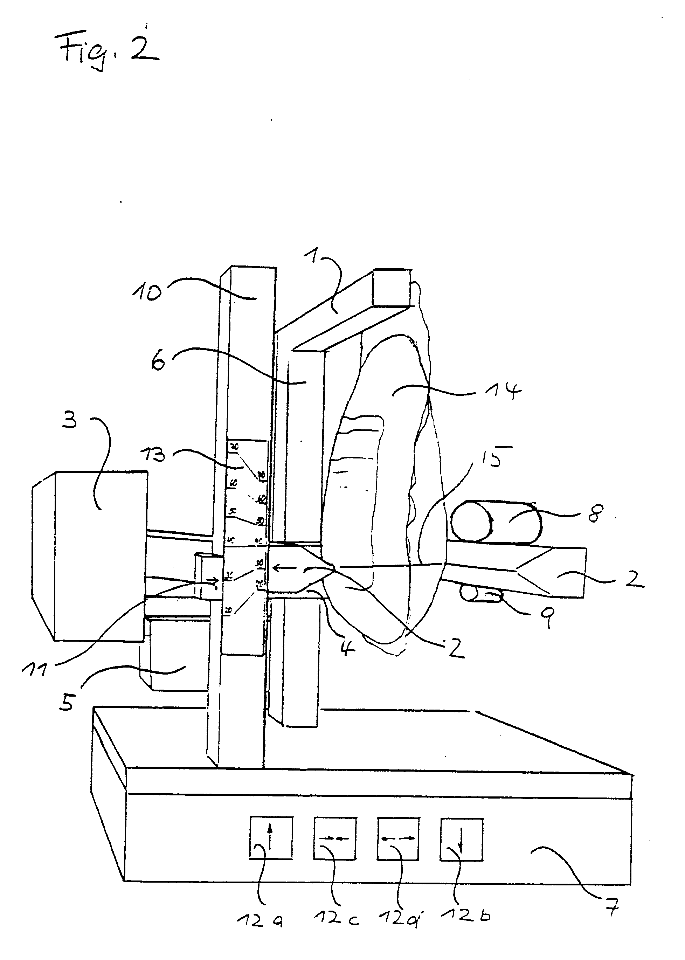

In Fig. 2 haben die Positionen 1 bis 9, 12, und 14 die gleiche Bedeutung wie in Fig. 1. Darüberhinaus enthält die in Fig. 2 gezeigte Vorrichtung einen Träger (10) mit einem Nomogramm (13). An dem Träger befindet sich ein verschiebbarer Endschalter (11). Die neben dem Träger (10) sich befindlichen Trennbacken (2) und der Endschalter (11) tragen jeweils einen Pfeil, der die genaue Einstellung der korrespondierenden Werte von Höhe der Grenzschicht (15) und der Höhe, in der die Trennbacken (2) zusammenfahren müssen, ermöglicht.

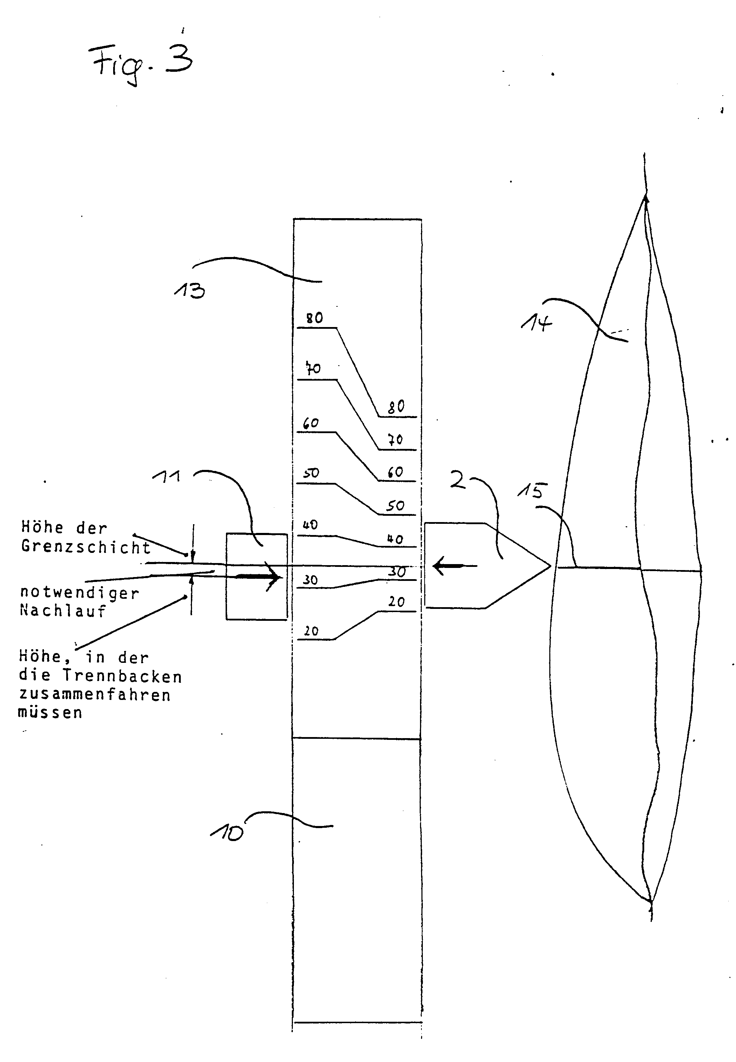

Fig. 3 zeigt eine vergrößerte Darstellung des Trägers (10) mit Nomogramm (13), einer Trennbacke (2), eines Endschalters (11), eines Beutels - (14) und der Grenzschicht (15). Etwas deutlicher als in Fig. 2 ist hier die Funktion des Nomogramms (13) zu sehen, auf dem für eine gegebene Maßzahl in Abhängigkeit von Beutelgröße und Füllvolumen die entsprechende Höhe, in der die Trennbacken zusammenfahren müssen, abgelesen werden kann.

Zur Sicherheit ist das Gerät mit einem (hier nicht gezeigten) Schutzgehäuse verkleidet. Durch eine Tür läßt sich ein Beutel im Gerät aufhängen bzw. wieder herausnehmen. Sobald die Tür geöffnet ist, erfolgt eine Abschaltung der Motoren.

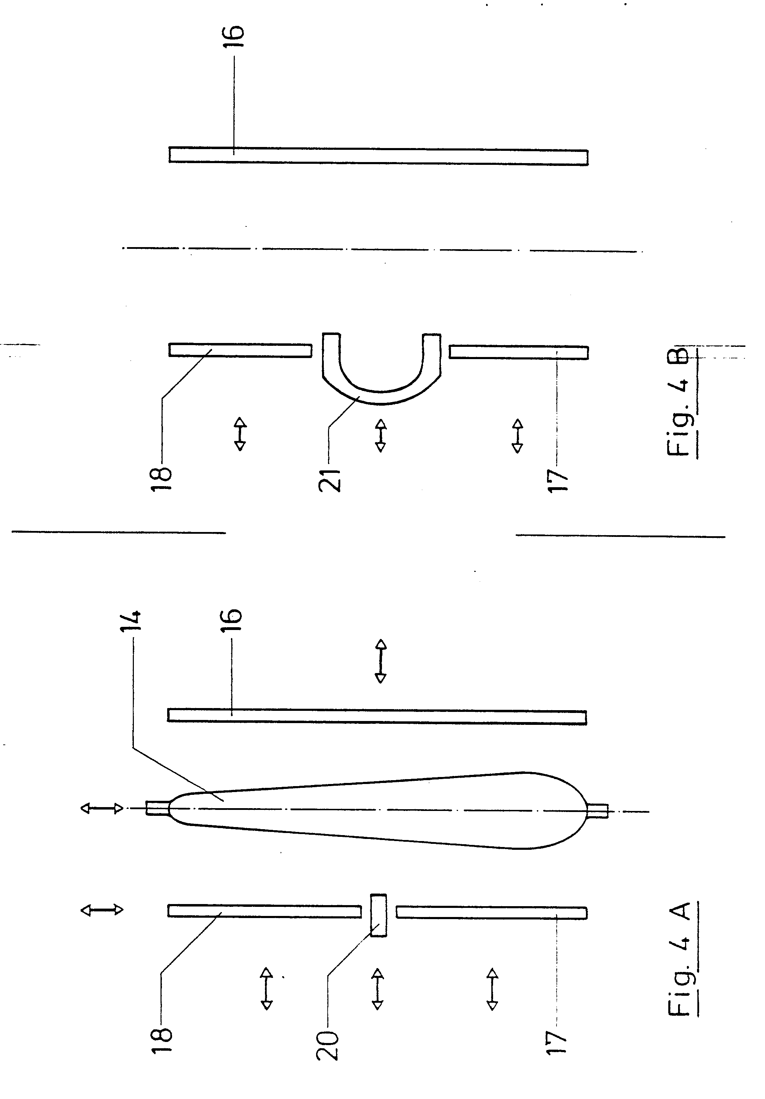

Fig. 4A zeigt eine starre Fläche (16) gegen die der Beutel (14) durch die Abpreßelemente (17) und (18) sowie das Trennelement (20) gepreßt werden, wobei sich sowohl die Fläche (16) als auch die Elemente (17, 18 und 20) gegeneinander bewegen können, und die waagerechten Pfeile die relative Bewegung der Einzelelemente gegenüber der Fläche (16)zeigen. Die Höhenplazierung des Beutels relativ zu den Elementen (17, 18, 20) ist variabel, angedeutet durch die senkrechten Pfeile, wobei die Höheneinstellung sowohl durch vertikale Bewegung des Beutels als auch durch vertikale Bewegung der Elemente (17, 18, 20) erfolgen kann. Es ist somit jeder Höhenbereich des Beutels für die Elemente (17, 18 und 20) ansteuerbar, wobei zunächst mit dem Trennelement (20) ein komplettes Abquetschen des Beutels in 2 Kompartimente erfolgt. Anschließend kann mit den Abpreßelementen (17) und (18) zusätzlich Druck auf die beiden Kompartimente des Beutels ausgeübt werden.

In Fig. 4B haben die Ziffern (16) bis (18), sowie die waagerechten Pfeile die gleiche Bedeutung wie in Fig. 4A. Trennelement (21) stellt ein zweifaches Abquetschelement dar.

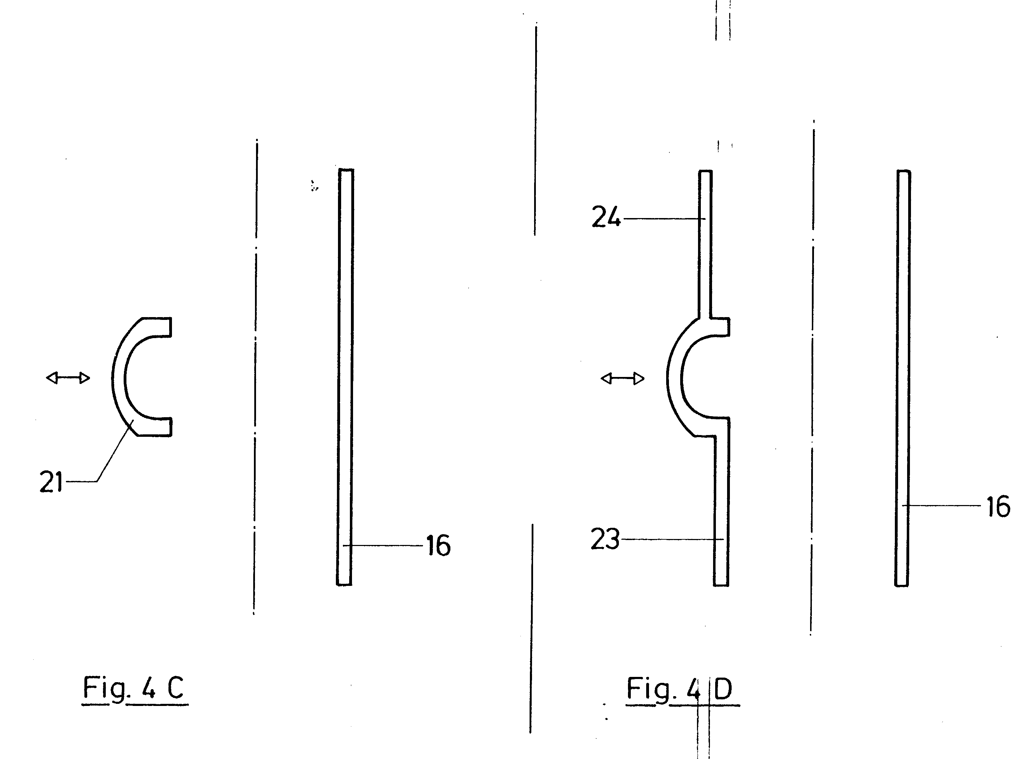

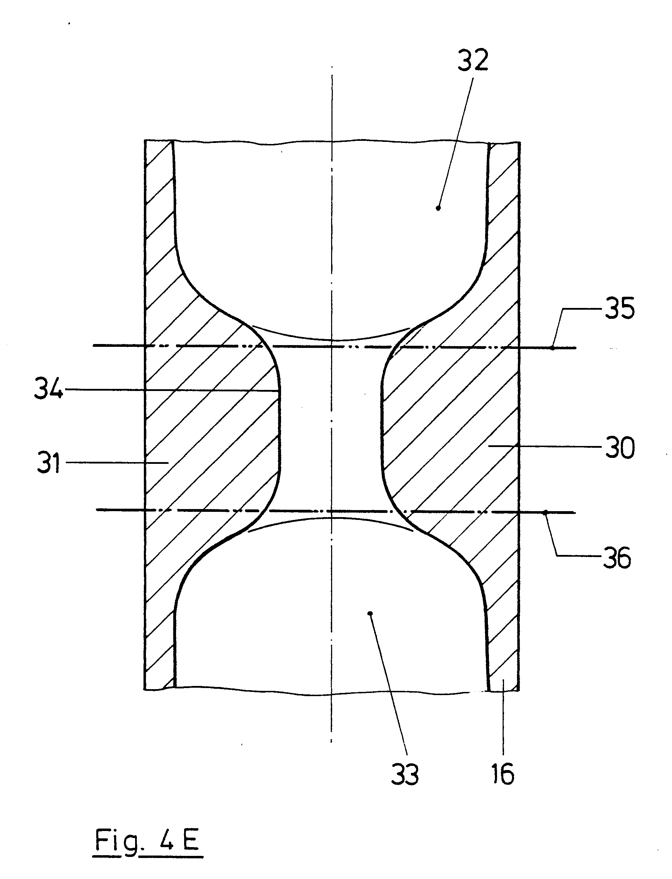

In Fig. 4C haben die Ziffern (16) und (21) sowie die waagerechten Pfeile die gleiche Bedeutung wie in Fig. 4B, jedoch fehlen hier die Abpreßelemente - (17) und (18).

Fig. 4D zeigt ein Abpreßelement, das nur aus einer zusammenhängenden Struktur besteht, wobei der Bogenbereich (22) zur Aufnahme eines bestimmten buffy coat-Volumens dient. Der Bereich - (23) dient zum Abpressen des Erythrozyten-Konzentrates, während Bereich (24) zum Abpressen des Plasmas dient. Wie hieraus ersichtlich, erfolgt in diesem Fall eine Abquetschung des buffy coats erst komplett, nachdem das Erythrozyten-Konzentrat aus dem unteren Bereich des Beutels entfernt wurde. Das überstehende Plasma wurde unter diesen Bedingungen noch nicht vollständig aus dem Beutel nach oben entfernt.

In den Ausführungsformen 4A bis 4D ist die starre Fläche (16) plan ausgebildet.

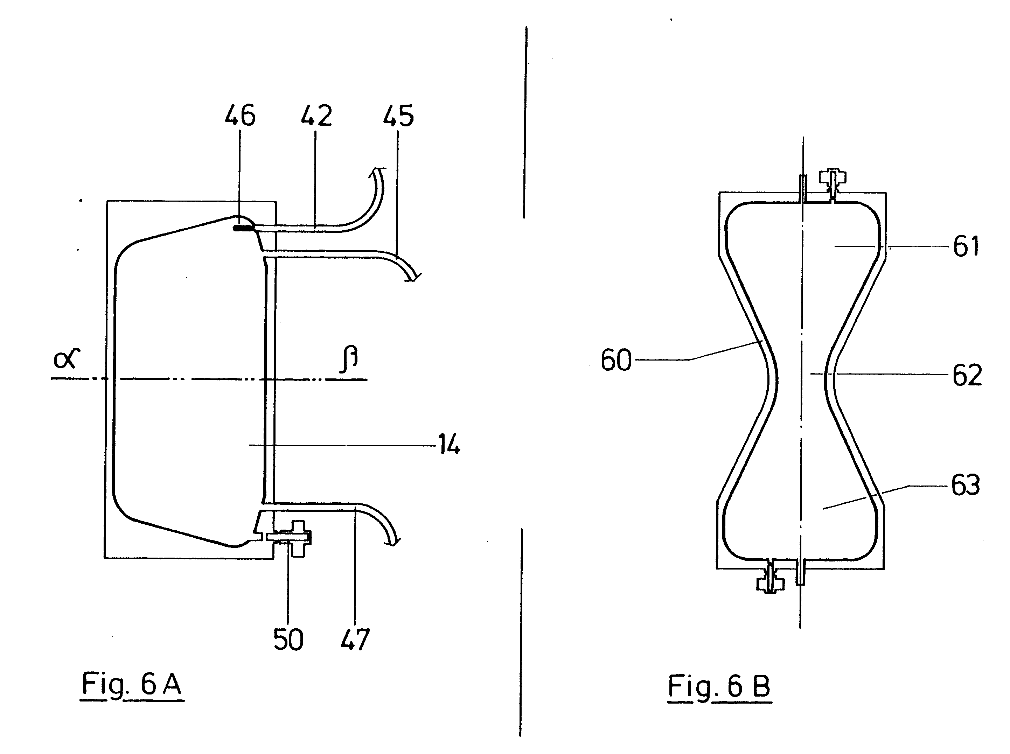

In Fig. 4E wird eine Ausführungsform in Aufsicht gezeigt, in der die starre Fläche (16) nicht plan ausgebildet ist. Die schraffierten Flächen (30) und (31) sind erhabene Flächen. Der zentrifugierte Beutel, insbesondere der Beutel gemäß Fig. 6B wird derart in die Fläche eingelegt, daß der Beutelkopf im Bereich (32) und das Beutelunterteil in den Bereich (33) zu liegen kommt.

Zwischen Kopf und Boden liegt eine Einschnürung (34). Durch diese "Wespentaillien"-Verengung läßt sich eine noch präzisere Abtrennung des buffy coats erreichen. Durch Abquetschelemente entlang der Linie (35) läßt sich das Erythrozytenkonzentrat, das sich im Beutelunterteil befindet von buffy coat und Plasma trennen. Durch Abquetschelemente entlang der Linien (35) und - (36) läßt sich außerdem der buffy coat-Bereich präzise abtrennen, so daß ohne besondere präparative Sorgfalt, z.B. durch Schwerkraft oder äußeren Druck das Erythrozytenkonzentrat aus dem Bereich (33) sehr schnell entfernt werden kann bzw. das Plasma aus dem Bereich (32) sehr - schnell abgetrennt werden kann, ohne daß mit Durchmischung mit dem Plasma gerechnet werden muß.

Bei der Anwendung von äußerem Druck müssen die beweglichen Abpreßelemente so ausgebildet sein, daß sie in die nicht erhabenen Bereiche (32) bzw. (33) passen und somit selektiv äußeren Druck auf Beutelkopf und Bodenbereich ausüben können.

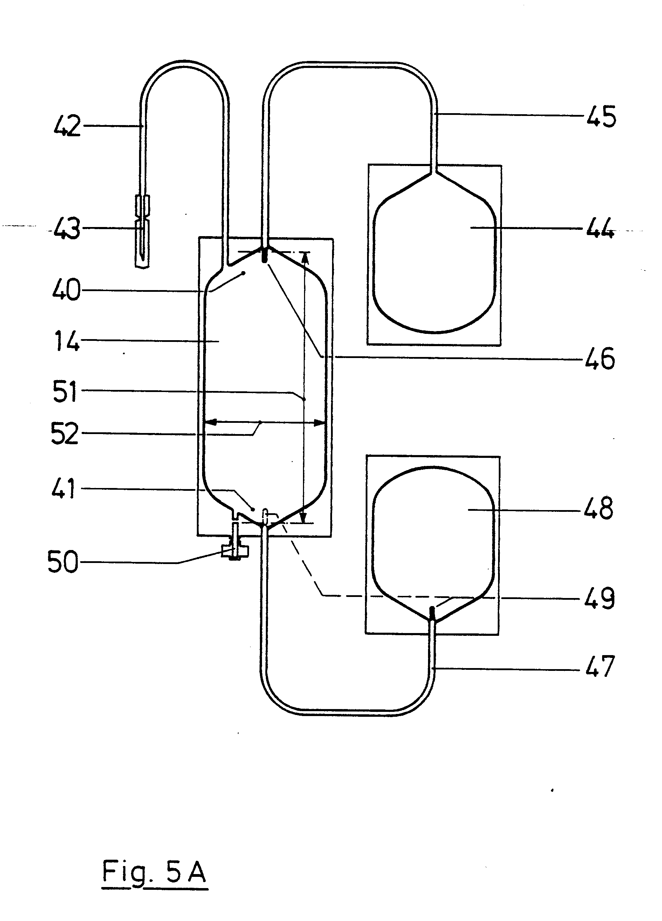

Das in Fig. 5A gezeigte Beutelsystem eignet sich besonders gut zur Gewinnung von drei Komponenten, insbesondere zur Gewinnung eines reinen Erythrozytenkonzentrats.

Der Blutbeutel (14) enthält einen Kopf (40) und Boden (41). Im Kopf befindet sich der Zuleitungsschlauch (42) mit der Blutbeutelkanüle (43), Beutel (44) ist mit Beutel (14) über einen Überleitungsschlauch (45) verbunden, Schlauch (45) ist am Kopf des Beutels (14) mit einem Abbrechtei (46) verschlossen. Der Boden des Beutels (14) ist über einen zweiten Überleitungsschlauch (47) mit einem Beutel (48) verbunden. Ein Abbrechteil (49) befindet sich am Abschluß des Schlauches (47) in dem Beutel (48) oder am Boden des Beutels (14). Beutel (14) enthält am Bodenbereich (41) noch einen Einstechstutzen (50). Längs-(51) und Schmalseite (52) des Beutels sind so ausgelegt, daß das Verhältnis mindestens 2 : 1 beträgt. Vorzugsweise sollte das Verhältnis 2 : 1 übersteigen. Die Trennung von Blutkomponenten ist um so besser je größer das Verhältnis ist, d.h. je schlanker die Säule ist, in dem die Blutkomponenten aufgetrennt werden. Aus Praktikabilitätsgründen wie Zentrifugation und Handhabung in den Blutpräparationslabors wird man ein Verhältnis von ca. 3 : 1 bis 2 : 1 anstreben.

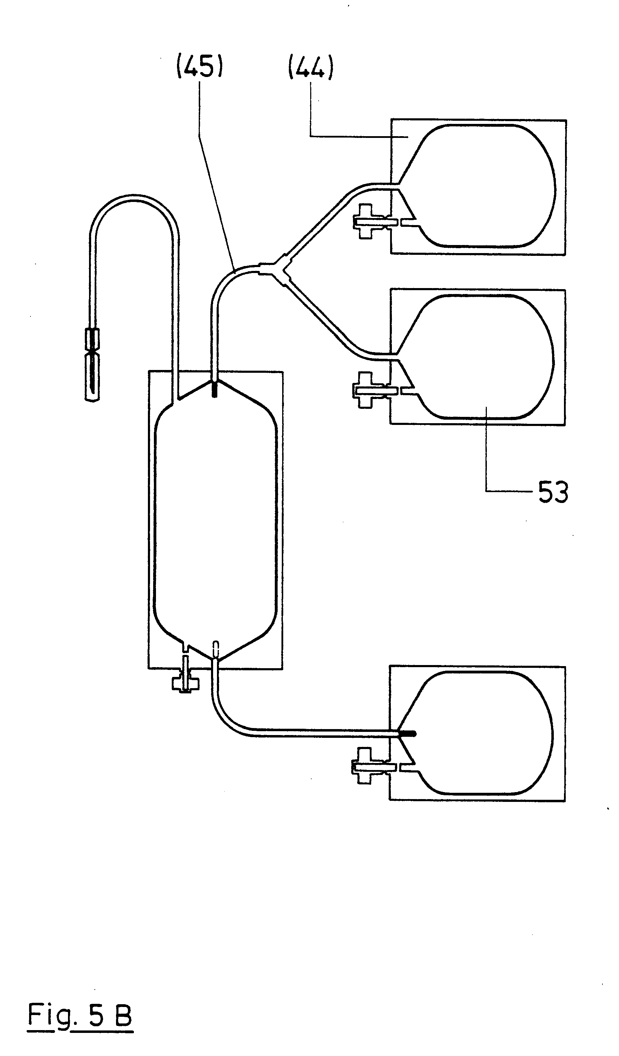

Fig. 5B zeigt ein Beutelsystem, das mit der Fig. 5A identische Teile (14) und (40) bis (52) aufweist und sich lediglich dadurch vom System der Fig. 5A unterscheidet, daß sich neben dem Beutel (44) ein über ein Y-Stück mit dem Schlauch (45) verbundener zusätzlicher Beutel (53) befindet, der z.B. eine Thrombozytenkonservierungslösung enthält.

Fig. 6A zeigt eine zweckmäßige Ausführungsform eines Blutbeutels (14) zur Verwendung in einem der vorstehend beschriebenen Beutelsysteme, bei dem am Kopf und am Boden des Beutels die entsprechenden Zuleitungs-bzw. Überleitungsschläuche (42), (45) und (47) seitlich angeordnet sind. Diese Schläuche befinden sich vorzugsweise nur auf einer Seite des Beutels.

Einstechstutzen (50) und Überleitungsschlauch (47) befinden sich am Boden des Beutels, Blutentnahmeschlauch (45) und Überleitungsschlauch (42) mit Abbrechteil (46) am Kopf des Beutels. Dieser Beutel läßt sich auf eine andere Weise zentrifugieren wie bisher übliche Beutel. Wird nämlich dieser Beutel entlang der Achse $\alpha$-$\beta$ gerollt oder gefaltet, so befinden sich Schläuche und Stutzen auf einer Beutelseite. Dieser gerollte und gefaltete Beutel kann dann derart in einen Zentrifugenbecher ein gesetzt werden, daß sich die Seite $\alpha$ am Becherboden und Seite $\beta$ am Becherkopf befindet. Diese Art der Zentrifugation kann dann vorteilhaft sein, wenn, wie bei der Präparation von Thrombozytenkonzentraten nur noch kleine Flüssigkeitsvolumina von ca. 50 ml vorliegen. Durch die Faltung oder Rollung des Beutels entlang der Achse $\alpha$-$\beta$ kann der Beutel ohne zusätzliche Halterung oder Stabilisierung in einem Standard-Blutbeutelbecher zentrifugiert werden.

Fig. 6B zeigt einen Blutbeutel (60) mit sogenannter Wespentaillenkonfiguration, der sich besonders zur Verwendung im Abquetschsystem gemäß Fig. 4E eignet. Der Beutel ist so konstruiert, daß er nicht erst durch Zusammenpressen im System gemäß Fig. 4E seine Wespentaillenform erhält, sondern von vornherein einen breiten Beutelkopf (61) und ein breites Beutelunterteil (63) enthält, die in dem Bereich (32) und (33) des Systems der Fig. 4E zu liegen kommen, sowie einen verengten Mittelbereich (62) enthält, der in der Einschnürung (34) des Systems der Fig. 4E zu liegen kommt.

Kopf und Boden des Blutbeutels (14) sollte abgerundet sein, was Abtrennung und Ausbeute des Plasmas (Obenabgang) und des Ery-Konzentrates (Untenabgang) begünstigt. Der Blutbeutel - (14) enthält zweckmäßigerweise eine Blutstabilisatorlösung, die ein Gerinnen des Blutes verhindert. Vorzugsweise enthält diese Lösung Glucose als Nährstoff für die Erythrozyten, bis das Erythrozyten-Konzentrat in den Beutel (48) Fig. 5 übergeleitet wird. Beutel (48) enthält die speziell zur Langzeitlagerung von Erythrozyten-Konzentraten erforderliche Erythrozyten-Konservierungslösung, z.B. wie in der DE-OS 32 25 408 beschrieben. Beutel (44) Fig. 5 dient zur Aufbewahrung des Plasmas. Dieses Plasma wird meist tiefgefroren. Hierfür kann ein Spezial-Tiefgefrierbeutel eingesetzt werden, wie z.B. in DEG 85 25 524.4 (Aufreißbeutel) beschrieben.

Nach Abtrennung von Plasma und Erythrozyten verbleibt das buffy coat, wie oben beschrieben, im Beutel (14). Von Pietersz et al, Vox Sang. 49 - (1985), ist ein Verfahren beschrieben, bei dem Thrombozytenkonzentrate aus buffy coats hergestellt werden. Mit Hilfe der Beutelausführung von Fig. 5B ist es möglich, entsprechend diesem Verfahren Thrombozytenkonzentrate herzustellen. Vorzugsweise wird das Thrombozytenkonzentrat im Beutel (14) gewonnen durch Suspendierung des buffy coats mit Plasma aus Beutel (44), gefolgt von einer "weichen", (d.h. mit geringen g-Zahlen) Zentrifugation, wobei restliche Erythrozyten bzw. Aggregate nach dem erfindungsgemäßen Verfahren nach unten abgequetscht und entfernt werden.

Das im Beutel (14) verbleibende thrombozytenreiche Plasma kann durch "harte" (dies mit höheren g-Zahlen) Zentrifugation zum Thrombozytenkonzentrat weiter angereichert werden, wobei das überstehende Plasma wieder in Beutel (44) transferiert wird. Aus Beutel (53) wird anschließend eine Thrombozytenkonservierungslösung in den Beutel (14) gegeben. Die Thrombozyten werden in dieser Lösung suspendiert und in Beutel (14) oder Beutel (53) zusammen mit dieser Konservierungslösung vorzugsweise durch kontinuierliche Durchmischung aufbewahrt. Für eine mehrtägige Lagerung sollte der Aufbewahrungsbeutel für Thrombozytenkonzentrate einen guten Gasaustausch gewährleisten, der für eine Lagerung von Thrombokonzentraten während mindestens 5 Tagen erforderlich ist.

Beutel (14) hat durch das große Volumen außerdem den Vorteil, daß er z.B. auch zur Aufnahme eines Pools von z.B. 5 -6 Konzentraten geeignet ist und nach Poolen sich (im erfindungsgemäßen Verfahren) diese Konzentrate zur Abtrennung von restlichen Kontaminationen von Zellen (Unterabgang) bzw. überstehender Suspensionsbzw. Plasmalösung (Obenabgang) besonders eignen.

**Ansprüche**

1. Verfahren zur Gewinnung von Komponenten aus einer Flüssigkeit in der die Komponenten in mindestens zwei räumlich getrennten Bereichen vorliegen, bei dem man einen mit dieser Mehrkomponenten-Flüssigkeit gefüllten durchsichtigen Beutel zwischen mindestens ein Trennelement hängt und das Trennelement in eine Höhe mit der Grenzschicht bringt und anschließend in Abquetschposition bringt und die Beutelfüllung in mindestens zwei getrennte flüssigkeitsdichte Volumina teilt, **dadurch gekennzeichnet**, daß man eine Maßzahl, die die Höhe der Grenzschicht angibt, ermittelt, die Höhe des Abquetschens mit Hilfe des Trennelementes anhand der ermittelten Maßzahl der Höhe der Grenzschicht im Beutel sowie der Beutelgröße und dessen Füllvolumen ermittelt, die Höheneinstellung des Trennelementes oder des Beutels mit Hilfe einer Energiequelle vornimmt und dann mit Hilfe einer Energiequelle das Trennelement in Abquetschposition bringt und nach dem Abquetschen die Komponenten voneinander trennt.

2. Verfahren nach Anspruch 1, dadurch gekennzeichnet, daß man die Maßzahl visuell durch Nachfahren des Trennelementes ermittelt.

3. Verfahren nach Anspruch 1, dadurch gekennzeichnet, daß man die Maßzahl durch Nachfahren eines Lichtzeigers ermittelt.

4. Verfahren nach Anspruch 1, dadurch gekennzeichnet, daß man die Maßzahl mit Hilfe eines optischen Detektors ermittelt.

5. Verfahren nach einem der vorstehenden Ansprüche, dadurch gekennzeichnet, daß man die Höhe der Abquetschposition des Trennelementes aufgrund der Maßzahl anhand von Tabellen ermittelt.

6. Verfahren nach einem der vorstehenden Ansprüche, dadurch gekennzeichnet, daß man die Höhe der Abquetschposition des Trennelementes aufgrund der Maßzahl mit Hilfe eines Nomogramms ermittelt.

7. Verfahren nach einem der vorstehenden Ansprüche, dadurch gekennzeichnet, daß man die Höhe der Abquetschposition des Trennelementes durch Einstellen eines verschiebbaren Endschalters festlegt.

8. Verfahren nach einem der vorstehende Ansprüche, dadurch gekennzeichnet, daß die Einstellung der Höhe der Abquetschposition des Trennelementes automatisch aus der Maßzahl der Höhe der Grenzschicht erfolgt.

9. Verfahren nach einem der vorstehenden Ansprüche, dadurch gekennzeichnet, daß man als Trennelement mindestens ein in der Höhe verstellbares Paar Trennbacken verwendet und diese durch Zusammenfahren in Abquetschposition bringt.

10. Verfahren nach einem der Ansprüche 1 bis 8, dadurch gekennzeichnet, daß die Höheneinstellung durch vertikale Bewegung des Beutels oder des Trennelementes erfolgt und man als Trennelement ein einfaches oder zweifaches Abquetschelement verwendet und dieses durch horizontale Bewegung in Richtung auf eine hinter dem Beutel sich befindliche starre Fläche in Abquetschposition bringt.

11. Verfahren nach einem der vorstehenden Ansprüche, dadurch gekennzeichnet, daß man den Beutel nach bzw. während des Abquetschens zusätzlich abpreßt.

12. Verfahren nach einem der vorstehenden Ansprüche, dadurch gekennzeichnet, daß man Komponenten aus einer Flüssigkeit gewinnt, in der die Komponenten in drei räumlich getrennten Bereichen vorliegen.

13. Vorrichtung zur Durchführung des Verfahrens nach einem der Ansprüche 1 bis 12, enthaltend ein in der Höhe verstellbares Trenngestell mit horizontal bewegbarem Trennelement und eine Aufhängung für einen mit einer Mehrkomponenten-Flüssigkeit gefüllten Beutel, gekennzeichnet durch einen Motor (5) zur Höheneinstellung der Trennelemente (2) oder des Beutels (4), einen Motor (3) zum In-Abquetschposition-Bringen der Trennelemente (2, 20, 21) und ein Steuergerät (7) für die Motoren (3, 5).

14. Vorrichtung nach Anspruch 13, gekennzeichnet durch einen Träger (10) für eine Meßskala (13).

15. Vorrichtung nach Anspruch 14, dadurch gekennzeichnet, daß die Meßskala (13) ein Nomogramm ist.

16. Vorrichtung nach einem der Ansprüche 13 bis 15, gekennzeichnet durch eine optische Ermittlungseinrichtung (8) der Grenzschicht.

17. Vorrichtung nach Anspruch 16, dadurch gekennzeichnet daß die optische Ermittlungseinrichtung aus einer Lichtquelle (8) und einem optischen Detektor (9) besteht.

18. Vorrichtung nach einem der Ansprüche 13 bis 17, gekennzeichnet durch einen verschiebbaren Endschalter (11).

19. Vorrichtung nach einem der Ansprüche 13 bis 18, gekennzeichnet durch eine starre Fläche - (16).

20. Vorrichtung nach Anspruch 19, zur Verwendung des Beutels gemäß Anspruch 23, dadurch gekennzeichnet daß die starre Fläche (16) eine wespentaillenförmige Aussparung (32, 33, 34) aufweist.

21. Vorrichtung nach einem der Ansprüche 13 bis 20, dadurch gekennzeichnet, daß sie zusätzlich Abpreßelemente (17, 18, 23, 24) aufweist.

22. Beutel, insbesondere Blutbeutel, zur Durchführung des Verfahrens nach einem der Ansprüche 1 bis 12, mit Zuleitungs-bzw. Überleitungsschläuchen am Kopf und Boden des Beutels, dadurch gekennzeichnet, daß Blutentnahmeschlauch (45) und Überleitungsschlauch (42) sich seitlich am Kopf des Beutels und Überleitungsschlauch (47) sich seitlich am Boden des Beutels befinden.

23. Beutel, insbesondere Blutbeutel, zur Durchführung des Verfahrens nach einem der Ansprüche 1 bis 12, gekennzeichnet durch eine Wespentaillenkonfiguration (61, 62, 63).

Fig. 1

Fig. 2

Fig. 3

13

80
70
60
50
40
30
20

11

Höhe der
Grenzschicht

notwendiger
Nachlauf

Höhe, in der
die Trennbacken
zusammenfahren
müssen

80
70
60
50
40
30
20

2

15

14

10

18

14

16

20

17

Fig. 4 A

18

16

21

17

Fig. 4 B

0 213 469

21

16

**Fig. 4 C**

24

23

16

**Fig. 4 D**

0 213 469

Fig. 4 E

Fig. 5 A

0 213 469

(45)   (44)

53

Fig. 5 B

Fig. 6B

Fig. 6A

63

62

60

61

47

50

71

β

α

45

42

46

0 213 469